# EUROPÄISCHE PATENTSCHRIFT

(11) **EP 0 634 912 B1**
(45) Veröffentlichungstag und Bekanntmachung des Hinweises auf die Patenterteilung: **19.11.1997**
(21) Anmeldenummer: 94906208.7
(22) Anmeldetag: 02.02.1994
(51) Int. Cl.: A61B 17/60

(54) **VORRICHTUNG ZUR WIRBELSÄULEN-VERSTEIFUNG UND/ODER -KORREKTUR**
DEVICE FOR STIFFENING AND/OR CORRECTING THE SPINE
DISPOSITIF PERMETTANT DE RAIDIR ET/OU DE CORRIGER LA COLONNE VERTEBRALE

(30) Priorität: 09.02.1993 DE 4303770
(43) Veröffentlichungstag der Anmeldung: 25.01.1995
(73) Patentinhaber: PLUS ENDOPROTHETIK AG, 6343 Rotkreuz (CH)
(72) Erfinder: GROB, Dieter, CH-8008 Zürich (CH)
(74) Vertreter: Popp, Eugen, Dr.
(86) Internationale Anmeldenummer: EP9400302
(87) Internationale Veröffentlichungsnummer: WO9417745

(56) Entgegenhaltungen:
- EP-A- 0 328 883
- EP-A- 0 383 992
- EP-A- 0 452 792
- WO-A-87/07134
- DE-A- 3 306 657
- GB-A- 2 252 732
- GB-A- 2 254 394

## Beschreibung

Die Erfindung betrifft eine Vorrichtung zur Versteifung und/oder Korrektur eines aus wenigstens zwei Wirbeln bestehenden Wirbelsäulenabschnitts gemäß dem Oberbegriff des Anspruchs 1.

Derartige Vorrichtungen zur Versteifung und/oder Korrektur eines aus wenigstens zwei Wirbeln bestehenden Wirbelsäulenabschnitts sind im Stand der Technik bekannt. Diese Vorrichtungen umfassen jeweils mindestens zwei schraubenförmige Halteeinrichtungen, die entsprechend an einem der Wirbel des Wirbelsäulenabschnitts befestigbar sind. Die Halteeinrichtungen sind zur Aufnahme und Festlegung eines Kompressions- bzw. Distraktionsstabes vorgesehen, welcher die Halteeinrichtungen miteinander verbindet. Auf diese Weise soll eine Versteifung bzw. eine sog. Spondylodese einzelner Wirbelsäulenabschnitte und/oder Korrektur bei Wirbelsäulenverkrümmung (Skoliose, Kyphose), bei Fehlstellungen (Rotation), bei Verletzung (Trauma), bei Neubildungen (Tumor) und vor allem bei Verschleiß bzw. degenerativer Wirbelsäulenerkrankung nach dem Prinzip einer möglichst rigiden, d.h. mechanisch ausgesprochen stabilen Osteosynthese, erreicht werden.

Als Halteeinrichtungen sind bei diesen Vorrichtungen in der Regel Schrauben bzw. sog. Pedikelschrauben wie auch an sich bekannte Haken vorgesehen, die über einen mechanisch stabilen Kompressions- bzw. Distraktionsstab miteinander verbindbar sind. Als ausgesprochen nachteilig bei diesen Vorrichtungen hat sich dabei die konstruktive Ausgestaltung der Verbindung zwischen den jeweiligen Halteeinrichtungen und dem Kompressions- bzw. Distraktionsstab herausgestellt. Einerseits weist die Verbindung dieser Vorrichtungen zumeist eine verhältnismäßig große Bauhöhe auf, welche die Verheilung nach dem operativen Eingriff sehr verlangsamt. Andererseits sind bei der Verbindung dieser Vorrichtungen vielfach Probleme mechanischer Art vorhanden, wodurch eine dauerhafte Fixierung zum einen und die Möglichkeit zur nachträglichen Positionskorrektur dieser Vorrichtungen zum anderen ausgeschlossen sind. Von weiterem Nachteil bei diesen Vorrichtungen sind deren fehlende, zumindest aber geringe Variationsmöglichkeiten hinsichtlich einer Fixation über eine geringe und sogleich große Länge. Vielmehr unterscheidet man zwischen Vorrichtungen zur Fixation, die zum einen ausschließlich für geringe Längen und zum anderen ausschließlich für große Längen vorgesehen sind, wobei ein Austausch untereinander und somit eine vielseitige Verwendung dieser Vorrichtungen nicht möglich sind. Darüber hinaus läßt sich mit diesen Vorrichtungen nicht zuletzt aufgrund ihrer Konstruktion eine nur sehr beschränkte Kraft zur Korrektur von Fehlstellungen des jeweiligen Wirbelsäulenabschnitts aufbringen. Schließlich sind diese Vorrichtungen durchweg auch verhältnismäßig teuer.

Eine Vorrichtung nach dem Oberbegriff des Anspruchs 1 ist zum Beispiel aus EP-A-328883 bekannt geworden

Der Erfindung liegt daher die Aufgabe zugrunde, eine Vorrichtung zur Versteifung und/oder Korrektur eines aus wenigstens zwei Wirbeln bestehenden Wirbelsäulenabschnitts der gattungsgemäßen Art bereitzustellen, die sämtliche aus dem Stand der Technik bekannten Nachteile beseitigt, insbesondere ausgesprochen kleinbauend ist, eine dauerhafte, nicht selbstlösende Fixierung und eine nachträgliche Positionskorrektur ermöglicht, vielseitig einsetzbar ist und eine hohe Kraftaufbringung gewährleistet.

Die Aufgabe wird durch die kennzeichnenden Merkmale des Anspruchs 1 gelöst.

Durch die erfindungsgemäße Ausgestaltung, daß nämlich der Kompressions- bzw. Distraktionsstab aus einem stabförmigen, von den Halteeinrichtungen aufnehmbaren Verbindungselement und wenigstens einem von dem stabförmigen Verbindungselement verschieblich aufnehmbaren, jeweils zwischen zwei einander benachbarten Halteeinrichtungen angeordneten und diese Halteeinrichtungen gegenseitig auf Abstand haltenden Distanzelement gebildet ist, wobei das Verbindungselement an einem Ende mit einem Gewinde und an dem anderen Ende mit ebenfalls einem Gewinde oder ggf. mit einer flanschartigen Verdickung od. dgl. zur Festlegung bzw. Verspannung von Halteeinrichtungen und Distanzelement versehen ist, sind verschiedene Vorteile gleichzeitig erreicht. So ist neben einer kompakten Bauweise eine dauerhafte Fixierung der erfindungsgemäßen Vorrichtung nach dem operativen Eingriff möglich, ohne daß sich die Halteeinrichtungen von dem Verbindungselement mit dem wenigstens einen Distanzelement des Kompressions- bzw. Distraktionsstabes insgesamt selbsttätig lösen. Des weiteren ist die Handhabung der erfindungsgemäßen Vorrichtung während des operativen Eingriffs ausgesprochen einfach gehalten, wobei die Vorrichtung gleichzeitig ohne Schwierigkeiten nachträglich einer Positionskorrektur von seiten des Operateurs unterzogen werden kann. Weiterhin läßt sich die erfindungsgemäße Vorrichtung vielseitig einsetzen, da deren Länge durch ein stabförmiges Verbindungselement verschiedener Länge und ein oder mehrere Distanzelemente unterschiedlicher Länge beliebig und somit an die jeweiligen räumlichen und körperspezifischen Gegebenheiten eines jeden Patienten angepaßt variierbar ist. Schließlich kann mit der Vorrichtung nach der Erfindung eine ausgesprochen hohe Kraft infolge einer unmittelbaren, postoperativen Stabilität an und zwischen den Wirbeln des Wirbelsäulenabschnitts ausgeübt werden, so daß die Reposition bzw. Korrektur einer Fehlstellung durch die Vorrichtung nach der Erfindung selbst erreichbar ist. In alternativer Ausgestaltung zu schraubenförmigen Halteeinrichtungen können ebensogut hakenförmige Halteeinrichtungen, wie im Stand der Technik bekannt, eingesetzt werden.

Vorteilhafte Ausgestaltungen der Erfindung sind in den Ansprüchen 2 bis 19 beschrieben.

Gemäß Anspruch 2 liegt es im Rahmen der Erfindung, daß die Stirnflächen der beiden Enden des wenigstens einen Distanzelements mit der zu der Längsachse des Distanzelements senkrechten Ebene zusammenfallen. Bei dieser Ausführungsform des Distanzelements handelt es sich somit um einen beidseitig geraden Hohlzylinder od. dgl., so daß sich die an das Distanzelement anschließenden Halteeinrichtungen genau senkrecht zu dem Distanzelement selbst und dem stabförmigen Verbindungselement des Kompressions- bzw. Distraktionsstabes erstrecken.

In alternativer Ausgestaltung fallen nach Merkmal des Anspruchs 3 eine der Stirnflächen der beiden Enden des wenigstens einen Distanzelements mit der zu der Längsachse des Distanzelements senkrechten Ebene und eine der Stirnflächen der beiden Enden des wenigstens einen Distanzelements mit einer von der zu der Längsachse des Distanzelements senkrechten Ebene verschiedenen Ebene zusammen. Bei dieser Ausführungsform des Distanzelements handelt es sich somit um einen einseitig geraden und einseitig schräg abgeschnittenen Hohlzylinder od. dgl., so daß sich die an das Distanzelement anschließenden Halteeinrichtungen zum einen genau senkrecht und zum anderen schräg in einem Winkel zu dem Distanzelement selbst und dem stabförmigen Verbindungselement des Kompressions- bzw. Distraktionsstabes erstrecken. Hierdurch können beispielsweise geringfügige Ungenauigkeiten bei der Einbringung einer der beiden Halteeinrichtungen in den Wirbel infolge einer nicht genau zentrierten Bohrung etc. nachträglich korrigiert und ausgegleichen werden. In entsprechender Weise ist ein genaues Justieren von Wirbeln des zu versteifenden bzw. zu korrigierenden Wirbelsäulenabschnitts zueinander während des operativen Eingriffs durch die Anordnung mehrerer Distanzelemente auf dem stabförmigen Verbindungselement des Kompressions- bzw. Distraktionsstabes mit unterschiedlich geformten Enden beliebig variierbar möglich.

Dementsprechend ist nach Anspruch 4 auch erfindungsgemäß vorgesehen, daß die Stirnflächen der beiden Enden des wenigstens einen Distanzelements mit einer zu der Längsachse des Distanzelements von der zu der Längsachse des Distanzelements senkrechten Ebene verschiedene Ebene zusammenfallen. Bei dieser Ausführungsform des Distanzelements handelt es sich somit um einen beidseitig schräg abgeschnittenen Hohlzylinder od. dgl., so daß die sich an das Distanzelement anschließenden Halteeinrichtungen jeweils schräg in entsprechendem Winkel zu dem Distanzelement selbst und dem stabförmigen Verbindungselement des Kompressions- bzw. Distraktionsstabes verlaufen. Insoweit ist die erfindungsgemäße Vorrichtung an beliebige anatomische Gegebenheiten des Patienten anpaßbar.

Vorteilhafterweise ist das Distanzelement gemäß Merkmal nach Anspruch 5 unterschiedlich lang ausgebildet, wodurch die Einsetzmöglichkeiten der erfindungsgemäßen Vorrichtung in Abhängigkeit von der Länge des stabförmigen Verbindungselements noch zusätzlich erhöht werden können.

Von besonderem Interesse für eine hohe Rotationsstabilität des wenigstens einen Distanzelelments in Verbindung mit den beiden benachbarten Halteeinrichtungen oder auch der Distanzelemente untereinander sind weiterhin die Merkmale der Ansprüche 6 und 7. Demnach sind die Stirnflächen jedes Distanzelements zur rotationsstabilen Anlage mit der oder den Halteeinrichtungen bzw. dem oder den benachbarten Distanzelementen aufgerauht ausgebildet. Insbesondere sind die Stirnflächen jedes Distanzelements zur rotationsstabilen Anlage mit etwa radial verlaufenden Nuten, Kerben od. dgl. und entsprechenden Vorsprüngen etc. versehen. Denkbar ist in diesem Zusammenhang auch durchaus, die Halteeinrichtungen in denjenigen Bereichen, welche mit den Stirnflächen des bzw. der Distanzelemente in Berührung bringbar sind, in gleicher Weise aufzurauhen oder mit derartigen radial verlaufenden Nuten, Kerben etc. sowie Vorsprüngen usw. zu versehen.

Um darüber hinaus die Handhabbarkeit der erfindungsgemäßen Vorrichtung noch weiter zu verbessern, weist das wenigstens eine Distanzelement gemäß Anspruch 8 einen Außenumfang mit einer Schlüsselfläche auf, mittels welcher das Distanzelement unter Zuhilfenahme eines Werkzeugs, insbesondere eines Schraubenschlüssels, bei der Montage um seine Längsachse verdrehbar ist. Dies hat zugleich den Vorteil, daß sich die Vorrichtung in Abhängigkeit von der Deformation bzw. Fehlstellung der Wirbel des auszurichtenden Wirbelsäulenabschnitts justieren bzw. positionsgenau korrigieren läßt. Ungeachtet dessen kann, sofern wenigstens das eine Distanzelement mindestens ein schräg abgeschnittenes Ende in bezug auf dessen Längsachse besitzt, zudem die aufzubringende Verspannungskraft, d.h. Klemmkraft, beim operativen Eingriff leicht, schnell und präzise eingestellt werden.

Von wesentlicher Bedeutung für eine vielseitige Verwendung der Vorrichtung nach der Erfindung sind weiterhin die Merkmale des Anspruchs 9. So kann ein weiteres stabförmiges, von Halteeinrichtungen aufnehmbares Verbindungselement vorgesehen sein, das wenigstens teilweise und im wesentlichen parallel zu dem einen stabförmigen, von den Halteeinrichtungen aufnehmbaren Verbindungselement verlaufend anordnenbar und zu letzterem relativ festlegbar ist. Infolge einer solchen zusätzlichen Fixationsmöglichkeit des Kompressions- bzw. Distraktionsstabes kann die Stabilität der erfindungsgemäßen Vorrichtung und damit zusammenhängend die stabile Verankerung des Wirbelsäulenabschnitts weiter erhöht werden.

Dies kann einerseits durch die Maßnahmen des Anspruchs 10 erreicht werden, daß nämlich an dem wenigstens einen Distanzelement ein weiteres, im wesentlichen parallel verlaufendes Distanzelement insbesondere durch Verschweißung unlösbar angebracht ist. Das weitere, im wesentlichen parallel verlaufende Distanzelement dient gleichzeitig der An- bzw. Verbindung zweier erfindungsgemäß zu montierender Vorrichtungen und damit der beliebigen Verlängerung bzw. Fortsetzung der einen oder eben der anderen bereits montierten Vorrichtung sowie dem räumlichen Versatz des gesamten Kompressions- bzw. Distraktionsstabes zur Anpassung dessen an die vorgegebenen anatomischen Besonderheiten des Patienten.

Alternativ zu der Ausführungsform der Vorrichtung nach Anspruch 10 kann entsprechend den Merkmalen der Ansprüche 11 und 12 erfindungsgemäß ebenso vorgesehen sein, daß die beiden stabförmigen, von den Halteeinrichtungen aufnehmbaren Verbindungselemente über Halteelemente miteinander verbunden sind, welche vorzugsweise zwei oder mehrere Ausnehmungen od. dgl. aufweisen, die der etwa spielfreien Aufnahme der beiden stabförmigen Verbindungselemente dienen.

Nach einem weiteren Merkmal der Erfindung gemäß Anspruch 13 umfassen die Halteeinrichtungen in vorteilhafter Weise jeweils einen Schraubenschaft mit einem Gewinde zum Einschrauben in den Wirbel und einen Schraubenkopf zur Aufnahme und Festlegung des stabförmigen Verbindungselements. Neben einer dauerhaften Fixierung der erfindungsgemäßen Vorrichtung zur Versteifung und/oder Korrektur eines aus wenigstens zwei Wirbeln bestehenden Wirbelsäulenabschnitts läßt sich durch diese Ausgestaltung eine besonders kleinbauende Konstruktion erhalten, mit der gleichzeitig große Kräfte übertragen werden können, so daß eine hohe Kraftaufbringung sichergestellt ist.

Von Vorteil zur zusätzlichen Vermeidung der Selbstlösung der gesamten Vorrichtung ist erfindungsgemäß entsprechend den Merkmalen des Anspruchs 14 vorgesehen, den Schraubenkopf der schraubenförmigen Halteeinrichtung mit einer Bohrung, vorzugsweise einer Mittelbohrung, zu versehen, in bzw. durch welche das stabförmige Verbindungselement im wesentlichen spielfrei durch Verschiebung in Richtung von dessen Längsachse eingesteckt werden kann.

In alternativer Ausgestaltung dazu ist nach den Merkmalen des Anspruchs 15 vorgesehen, daß der Schraubenkopf der schraubenförmigen Halteeinrichtung mit einem Längsschlitz, vorzugsweise einem Mittellängsschlitz, od. dgl. schlitzförmigen Ausnehmung versehen ist, in welche das stabförmige Verbindungselement etwa spielfrei durch seitliche Verschiebung eingebracht werden kann. Auf diese Weise vereinfacht sich die Handhabung der erfindungsgemäßen Vorrichtung während der Montage bzw. auch Demontage bei dem operativen Eingriff ungemein.

Um die Korrekturmöglichkeiten noch weiter zu verbessern, liegt es darüber hinaus nach Anspruch 16 im Rahmen der Erfindung, daß der Schraubenkopf und der Schraubenschaft der schraubenförmigen Halteeinrichtung über einen Quersteg und/oder über einen Längssteg miteinander verbunden sind.

In bevorzugter Ausgestaltung der Erfindung bestehen die Halteeinrichtung und/oder das stabförmige Verbindungselement und/oder die mit dem Verbindungselement zusammenwirkenden Muttern aus Metall, insbesondere aus einer Metallegierung. Als Metall bzw. eine Metallegierung bietet sich beispielsweise Titan oder eine Legierung hieraus an. Hierdurch wird die Verträglichkeit der erfindungsgemäßen Vorrichtung schon während des operativen Eingriffs und der sich hieran anschließenden Heilungsphase heraufgesetzt. Damit einhergehend wird eine hohe Operationsqualität erhalten. In diesem Zusammenhang ist das Material des stabförmigen Verbindungselements allerdings derart zu wählen, daß das stabförmige Verbindungselement einerseits eine hohe Biegesteifigkeit aufweist, andererseits aber für eine genaue Justierung und/oder präzise Positionskorrektur der erfindungsgemäßen Vorrichtung beim operativen Eingriff zumindest in gewissem Maße verbiegbar ist.

Entsprechend dem Merkmal des Anspruchs 18 besteht bzw. bestehen das bzw. die Distanzelement/e zur Heraufsetzung der Verträglichkeit der Vorrichtung ebenfalls aus Metall, insbesondere aus Titan oder einer Legierung hieraus.

Schließlich kann bzw. können das bzw. die Distanzelement/e alternativ bzw. kumulativ auch aus einem bedingt elastischen/flexiblen Material, wie z.B. Polyethylen, bestehen. Die erfindungsgemäße Vorrichtung kann insoweit einen Wirbelsäulenabschnitt bis zu einem vorbestimmten Grad, welcher von der Flexibilität und Elastizität des Materials sowie der Anordnung insgesamt abhängig ist, versteifen.

Weitere Merkmale, Vorteile und Einzelheiten der Erfindung ergeben sich aus der nachfolgenden Beschreibung einiger bevorzugter Ausführungsformen der Erfindung sowie anhand der Zeichnung. Hierbei zeigen:
- Fig. 1: eine Seitenansicht einer Ausführungsform einer erfindungsgemäß ausgebildeten Vorrichtung;
- Fig. 2: eine Seitenansicht einer weiteren Ausführungsform einer erfindungsgemäß ausgebildeten Vorrichtung ohne Darstellung der zweiten Halteeinrichtung mit Unterlegscheibe und Mutter;
- Fig. 3: eine Seitenansicht einer anderen Ausführungsform einer erfindungsgemäß ausgebildeten Vorrichtung;
- Fig. 4: eine Seitenansicht einer noch anderen Ausführungsform einer erfindungsgemäß ausgebildeten Vorrichtung;
- Fig. 5: ein erfindungsgemäßes Halteelement; und
- Fig. 6 bis 9: Vorder- bzw. Rückansichten mehrerer Ausführungsformen von erfindungsgemäß ausgebildeten, schraubenförmigen Halteeinrichtungen.

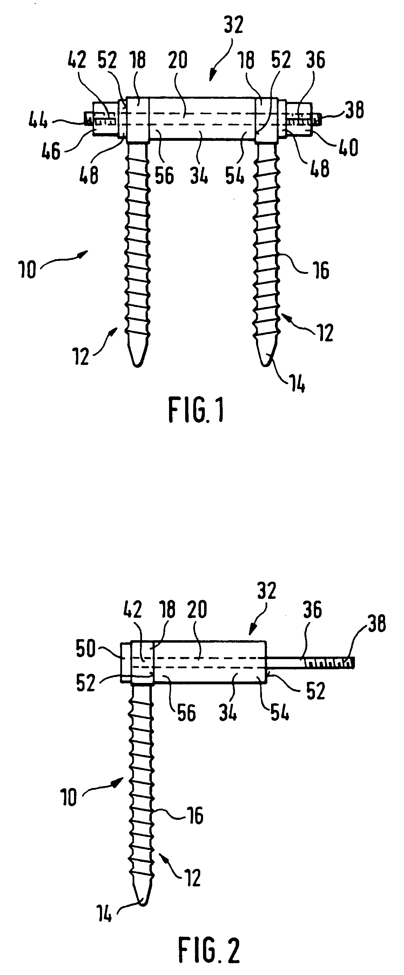

In Fig. 1 ist eine erste Ausfuhrungsform einer Vorrichtung 10 zur Versteifung und/oder Korrektur eines aus wenigstens zwei Wirbeln (nicht dargestellt) bestehenden Wirbelsäulenabschnitts gezeigt. Die Ausführungsform der Vorrichtung 10 nach Fig. 1 ist ebenso wie die weiteren Ausführungsformen der Vorrichtung 10 nach den Fig. 2 bis 4 zur dorsalen Implantation an der Lendenwirbelsäule, der Brustwirbelsäule und der unteren Halswirbelsäule sowie zur ventralen Implantation an der Lendenwirbelsäule und der mittleren bis unteren Brustwirbelsäule geeignet. Bei sowohl der dorsalen als auch ventralen Anwendung der Vorrichtung 10 kann die Fusion entweder in situ, mit oder ohne zusätzliche Eingriffe, wie z.B. Dekompression, Debridement etc., oder mit Stellungskorrektur, wie z.B. Skoliose, Kyphose, Rotation etc., erfolgen, wobei die Ätiologie der Deformität, wie z.B. Traume, ossäre Defekte, neurogene, muskuläre, kongenitale Deformitäten etc., unerheblich ist.

Die erste Ausführungsform der Vorrichtung 10 aus Fig. 1 umfaßt zwei schraubenförmige Halteeinrichtungen 12, die jeweils an einem der Wirbel des Wirbelsäulenabschnitts befestigbar sind. Insbesondere wird eine solche Halteeinrichtung 12 in bekannter Weise in den Pedikel, d.h. in den Wirbelbogen zwischen Dornfortsatz, Querfortsatz und dem entsprechenden oberen Gelenkfortsatz eingeschraubt.

Die schraubenförmigen Halteeinrichtungen 12 umfassen jeweils einen Schraubenschaft 14 mit einem Gewinde 16 zum Einschrauben in den Wirbel des entsprechenden Wirbelsäulenabschnitts. Darüber hinaus weisen die schraubenförmigen Halteeinrichtungen 12 jeweils einen Schraubenkopf 18 zur Aufnahme und Festlegung eines stabförmigen Verbindungselements 20 auf.

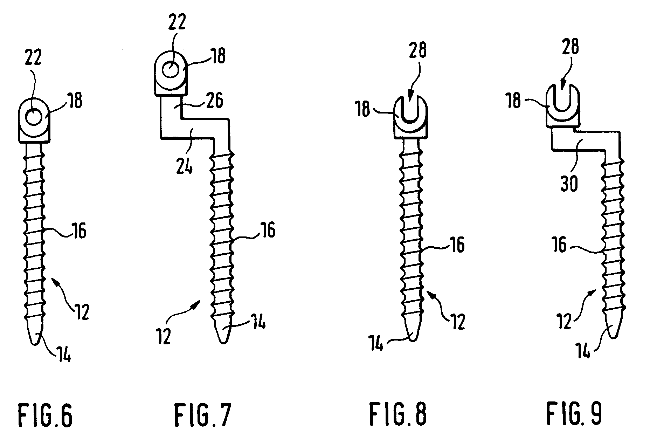

Zur etwa spielfreien Aufnahme des stabförmigen Verbindungselements 20 ist der Schraubenkopf 18 der schraubenförmigen Halteeinrichtung 12 - wie in den Fig. 6 und 7 gezeigt - mit einer Bohrung, vorzugsweise einer Mittelbohrung 22, versehen. Die schraubenförmige Haltevorrichtung 12 ist insoweit auf das stabförmige Verbindungselement 20 durch eine Verschiebebewegung in Richtung der Längsachse des Verbindungselements 20 bzw. der in den Schraubenkopf 18 vorgesehenen Mittelbohrung 22 aufsteckbar od. dgl.

Die Ausführungsform der schraubenförmigen Halteeinrichtung 12 gemäß Fig. 7 unterscheidet sich von derjenigen nach Fig. 6 lediglich dadurch, daß der Schraubenkopf 20 mit der Mittelbohrung 22 und der Schraubenschaft 14 der schraubenförmigen Halteeinrichtung 12 zusätzlich über einen Quersteg 24 und einen Längssteg 26 miteinander verbunden sind. Hierdurch wird ein weiterer Freiheitsgrad bei der Handhabung der erfindungsgemäßen Vorrichtung 10 erreicht, der eine genaue Justierung und ggf. nachträgliche Positionskorrketur für das Verbindungselement 20 zur Folge hat.

Zur im wesentlichen spielfreien Aufnahme des stabförmigen Verbindungselements 20 ist der Schraubenkopf 18 der schraubenförmigen Halteeinrichtung 12 alternativ - wie in den Fig. 8 und 9 dargestellt - mit einem Längsschlitz, vorzugsweise einem Mittellängsschlitz 28, od. dgl. schlitzförmigen Ausnehmung ausgestattet. Die schraubenförmige Halteeinrichtung 12 ist insofern auf das stabförmige Verbindungselement 20 durch eine seitliche Aufsetzbewegung od. dgl. bringbar, wodurch die Handhabbarkeit der schraubenförmigen Halteeinrichtung 12 während des operativen Eingriffs für den Operateur um ein wesentliches verbessert wird.

Die Ausführungsform der schraubenförmigen Halteeinrichtung 12 nach Fig. 9 differiert von derjenigen nach Fig. 8 nur dadurch, daß sich der Schraubenkopf 18 nicht unmittelbar an den Schraubenschaft 14 der schraubenförmigen Halteeinrichtung 12 anschließt, sondern vielmehr mit dem Schraubenschaft 14 über einen Quersteg 30 verbunden ist. Denkbar ist ebenso in diesem Zusammenhang, daß der Schraubenkopf 18 und der Schraubenschaft 14 über einen sich in Längsrichtung der schraubenförmigen Halteeinrichtung 12 erstreckenden Längssteg (nicht dargestellt) anstatt des Querstegs 30 in gegenseitiger Verbindung stehen. Der hieraus resultierende zusätzliche Freiheitsgrad erhöht die Genauigkeit bei der Justierung und ggf. nachträglichen Positionskorrektur der erfindungsgemäßen Vorrichtung 10 zur Versteifung und/oder Korrektur des Wirbelsäulenabschnitts um ein Vielfaches. Zudem läßt sich die Vorrichtung 10 nach der Erfindung flexibel an die besonderen anatomischen Gegebenheiten eines jeden Patienten anpassen, so daß die Verwendbarkeit der gesamten Vorrichtung 10 ausgesprochen vielseitig ist.

Die beiden Halteeinrichtungen 12 entsprechend Fig. 1 sind zur Aufnahme und Festlegung eines die beiden Halteeinrichtungen 12 miteinander verbindenden Kompressions- bzw. Distraktionsstabes 32 vorgesehen. Der Kompressions- bzw. Distraktionsstab 32 ist einerseits aus dem stabförmigen, von den Halteeinrichtungen 12 aufnehmbaren Verbindungselement 20 und andererseits aus wenigstens einem, d.h. hier ausschießlich einem Distanzelement 34 gebildet. Das Distanzelement 34 ist von dem stabförmigen Verbindungselement 20 entsprechend den Halteeinrichtungen 12 im wesentlichen spielfrei und verschieblich aufnehmbar. Weiterhin ist das Distanzelement 34 jeweils zwischen zwei einander benachbarten Halteeinrichtungen 12 angeordnet, um diese beiden Halteeinrichtungen 12 gegenseitig auf Abstand zu halten.

Wie aus den Fig. 1 und 2 deutlich hervorgeht, ist das Verbindungselement 20 an einem Ende 36 mit einem Gewinde 38 versehen, das mit einer Mutter 40 entsprechend Fig. 1 zusammenwirkt. An dem anderen Ende 42 ist das Verbindungselement 20 nach Fig. 1 ebenfalls mit einem Gewinde 44 ausgestattet, das mit einer Mutter 46 konterbar ist. Zwischen den beiden endseitig angeordneten Muttern 40, 46 und den diesen benachbarten Halteeinrichtungen 12 sind jeweils eine Unterlegscheibe 48, ein Sicherungsring od. dgl. auf das Verbindungselement 20 aufgebracht. Durch Anziehen der beiden Muttern 40, 46 werden folglich die beiden Halteeinrichtungen 12 und das zwischen den beiden Halteeinrichtungen 12 vorgesehene Distanzelement 34 miteinander festgelegt bzw. sogar gegeneinander verspannt.

Die weitere Ausführungsform der erfindungsgemäßen Vorrichtung 10 zur Versteifung und/oder Korrektur eines Wirbelsäulenabschnitts nach Fig. 2 unterscheidet sich von derjenigen nach Fig. 1 lediglich dadurch, daß das Verbindungselement 20 an dem einen Ende 36 mit einem Gewinde 38 und einer (nicht näher dargestellten) Mutter 40, jedoch an dem anderen Ende 42 mit einer flanschartigen Verdickung 50 od. dgl. anstelle des Gewindes 44 und der Mutter 46 ausgestattet ist. Insoweit werden hier die beiden Halteeinrichtungen 12 und das zwischen den beiden Halteeinrichtungen 12 eingefügte Distanzelement 34, die sämtlich von dem Verbindungselement 20 aufgenommen sind, durch Anziehen lediglich der einen (nicht dargestellten) Mutter zwischen der flanschartigen Verdickung 50 und eben der Mutter miteinander festgelegt bzw. gegenseitig verspannt. Einer selbsttätigen Lösung kann zusätzlich beispielsweise mittels einer in Fig. 2 nicht gezeigten Unterlegscheibe, Sicherungsring oder ähnlichem sicher entgegengewirkt werden. Alle übrigen Bauteile, die mit denjenigen der Ausführungsform der Vorrichtung 10 nach Fig. 1 übereinstimmen, sind mit gleichen Bezugsziffern versehen.

Wie aus den Fig. 1 und 2 weiterhin zu entnehmen ist, fallen die Stirnflächen 52 der beiden Enden 54, 56 des wenigstens einen, d.h. hier des einzigen Distanzelements 34 mit der Ebene zusammen, die zu der Längsachse des Distanzelements 34 bzw. auch zu der Blattebene senkrecht steht. Das Distanzelement 34 nach den Fig. 1 und 2 ist insofern als ein beidseitig gerader Hohlzylinder ausgebildet.

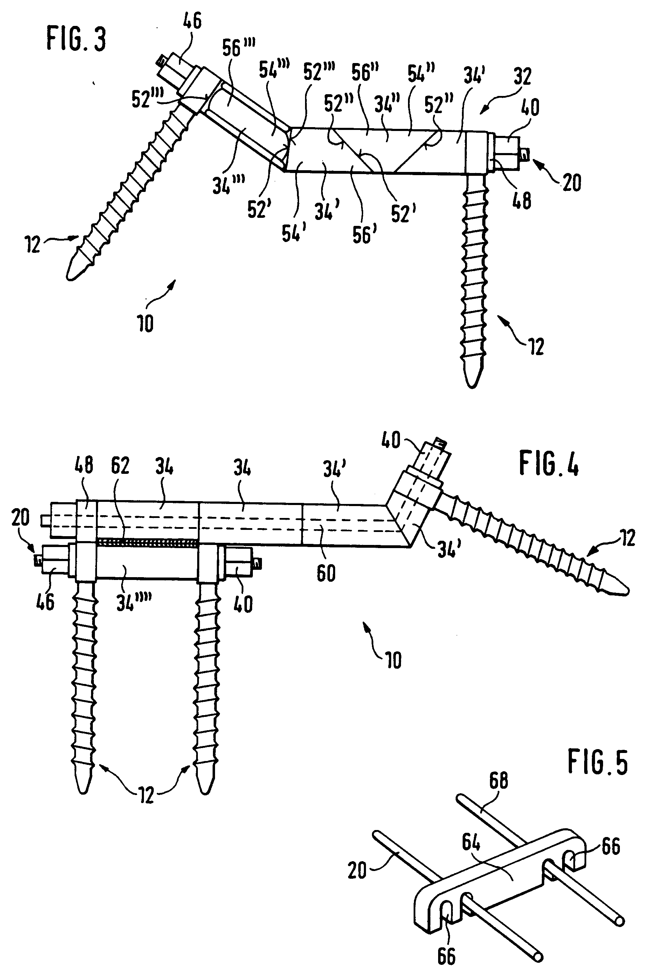

Die Ausführungsform der Vorrichtung 10 zur Versteifung und/oder Korrektur eines Wirbelsäulenabschnitts gemäß Fig. 3 unterscheidet sich von derjenigen nach Fig. 1 ausschließlich durch die besondere Ausgestaltung der einzelnen Distanzelemente 34', 34'' und 34'''. Alle übrigen Bauteile, die mit denjenigen der Ausführungsform der Vorrichtung 10 nach Fig. 1 übereinstimmen, sind mit gleichen Bezugsziffern versehen.

Demnach fällt bei den beiden Distanzelementen 34' in Fig. 3 eine der Stirnflächen 52' der beiden Enden 54', 56', nämlich hier des Endes 54', mit der Ebene zusammen, die senkrecht zu der Längsachse des Distanzelements 34 und zur Blattebene steht. Weiterhin liegt bei den beiden Distanzelementen 34' nach Fig. 3 eine der Stirnflächen 52' der beiden Enden 54', 56', nämlich hier des Endes 56', in einer Ebene, die von der zu der Längsachse des Distanzelements 34' senkrechten Ebene und damit auch zur Blattebene unterschiedlich ist. Das Distanzelement 34' ist daher quasi als ein einseitig gerader und ein einseitig schräg abgeschnittener Hohlzylinder ausgestaltet.

Ein weiteres Distanzelement 34'' weist gemäß Fig. 3 Stirnflächen 52'' an den beiden Enden 54'', 56'' auf, die mit einer Ebene zusammenfallen, welche mit einer von der zu der Längsachse des Distanzelements 34'' senkrechten Ebene und damit auch der Blattebene verschieden ist. Das Distanzelement 34'' ist somit als ein beidseitig schräg abgeschnittener Hohlzylinder od. dgl. ausgebildet.

Schließlich ist in Fig. 3 noch ein Distanzelelment 34''' abgebildet, das einen Außenumfang mit einer Schlüsselfläche oder ähnlichem aufweist, die zum Verdrehen des Distanzelements 34''' um seine Längsachse mittels eines Werkzeugs, insbesondere eines Schraubenschlüssels, vorgesehen ist. Im übrigen fallen eine der Stirnflächen 52''' der beiden Enden 54''', 56''' des Distanzelements 34''' mit der zu der Längsachse des Distanzelements 34''' senkrechen Ebene und eine der Stirnflächen 52''' der beiden Enden 54''', 56''' des Distanzelements 34 mit einer von der zu der Längsachse des Distanzelements 34''' senkrechten Ebene verschiedenen Ebene zusammen.

Entsprechend Fig. 3 ist das Verbindungselement 20 des Kompressions- bzw. Distraktionsstabes 32 durch die bestimmte Anordnung der Distanzelemente 34', 34'' und 34''' zueinander sowie deren spezielle Ausbildung an den Enden 54', 56', 54'', 56'' und 54''', 56''' in einer Richtung abgewinkelt bzw. gebogen. Durch Verdrehung der einzelnen Distanzelemente 34', 34'' und 34''' zueinander läßt sich zudem die Kompressions- bzw. Distraktionswirkung sowie die Verspannungskraft präzise einstellen.

Die Ausführungsform der Vorrichtung 10 zur Versteifung und/oder Korrektur eines Wirbelsäulenabschnitts nach Fig. 4 unterscheidet sich von derjenigen nach Fig. 1 im wesentlichen ebenfalls durch die spezielle Anordnung und Ausgestaltung der Distanzelemente 34, 34' und 34''''. Sämtliche Bauteile, die mit denjenigen der Ausführungsform der Vorrichtung 10 nach Fig. 1 übereinstimmen, sind gleichfalls mit identischen Bezugsziffern versehen.

Bei der Vorrichtung 10 gemäß Fig. 4 ist neben dem Verbindungselement 20, welches zwei schraubenförmige Halteeinrichtungen 12 miteinander verbindet, ein weiteres stabförmiges von Halteeinrichtungen 12 aufnehmbares Verbindungselement 60 vorgesehen. Das Verbindungselement 60 kann - wie in Fig. 4 verdeutlicht - wenigstens teilweise und im wesentlichen parallel zu dem Verbindungselement 20 verlaufend angeordnet und zu diesem relativ festgelegt werden. Zu diesem Zweck ist an dem wenigstens einen Distanzelement 34'''' ein weiteres, im wesentlichen parallel verlaufendes Distanzelement 34 unlösbar angebracht. Nach Fig. 4 sind das Distanzelement 34'''' und das Distanzelement 34 beispielsweise über eine Schweißnaht 62 miteinander verbunden.

In alternativer und/oder kumulativer Ausgestaltung der Erfindung können zwei stabförmige, von den Halteeinrichtungen 12 aufnehmbare Verbindungselemente 20, 68 - wie in Fig. 5 schematisch angedeutet - über Halteelemente 64 miteinander verbunden sein. Die Halteelemente 64 sind hierfür mit zwei oder mehreren schlitzförmigen od. dgl. Ausnehmungen 66 versehen, welche der im wesentlichen spielfreien Aufnahme der beiden stabförmigen Verbindungselemente 20, 60 dienen.

Entsprechend den Fig. 1 bis 4 können die Distanzelemente 34, 34', 34'', 34''' und 34'''' sämtlich unterschiedlich lang ausgebildet sein, wodurch eine flexible Verwendungsmöglichkeit der Vorrichtung 10 gegeben ist.

Zudem sind die Stirnflächen 52, 52', 52'', 52''' und 52'''' jedes Distanzelements 34, 34', 34'', 34''' und 34'''' zur rotationsstabilen Anlage mit der/den Halteeinrichtungen 12 und/oder dem/den benachbarten Distanzelement/en 34, 34', 34'', 34''' und 34'''' aufgerauht. Insbesondere sind die Stirnflächen 52, 52', 52'', 52''', 52'''' jedes Distanzelements 34, 34', 34'', 34''' und 34'''' mit in den Zeichnungen nicht näher dargestellten Nuten, Kerben od. dgl. sowie Vorsprüngen etc. versehen, welche in bezug auf die Längsachse der Distanzelemente 34, 34', 34'', 34''' und 34'''' etwa radial verlaufen.

Vorzugsweise sind die Halteeinrichtungen 12 und/oder das stabförmige Verbindungselement 20, 60, 68 und/oder die mit dem Verbindungselement 20, 60, 68 zusammenwirkenden Muttern 40, 46 aus Metall, insbesondere aus Titan oder einer Legierung hieraus, hergestellt. Gleiches gilt für das bzw. die Distanzelemente 34, 34', 34'', 34''' und 34'''', die alternativ hierzu auch aus einem bedingt elastischen/flexiblen Material, insbesondere Polyethylen, bestehen können.

Die vorliegende Erfindung ist nicht auf das bzw. die zuvor beschriebenen Ausführungsbeispiele beschränkt. Insbesondere ist es so denkbar, anstatt schraubenförmiger Halteeinrichtungen 12 hakenförmige Halteeinrichtungen (nicht dargestellt) einzusetzen, die auf herkömmliche Weise an den jeweiligen Wirbeln des zu operierenden Wirbelsäulenabschnitts befestigbar sind.

Sämtliche in den Anmeldungsunterlagen offenbarten Merkmale werden als erfindungswesentlich beansprucht, soweit sie einzeln oder in Kombination gegenüber dem Stand der Technik neu sind.

## Patentansprüche

1. Vorrichtung zur Versteifung und/oder Korrektur eines aus wenigstens zwei Wirbeln bestehenden Wirbelsäulenabschnitts, umfassend mindestens zwei schraubenförmige, jeweils an einem der Wirbel des Wirbelsäulenabschnitts befestigbare Halteeinrichtungen (12) zur Aufnahme und Festlegung eines die Halteeinrichtungen (12) miteinander verbindenden Kompressions- bzw. Distraktionsstabes (32),
**dadurch gekennzeichnet**,
daß der Kompressions- bzw. Distraktionsstab (32) aus einem stabförmigen, von den Halteeinrichtungen (12) aufnehmbaren Verbindungselement (20) und wenigstens einem von dem stabförmigen Verbindungselement (20) verschieblich aufnehmbaren, jeweils zwischen zwei einander benachbarten Halteeinrichtungen (12) angeordneten und diese Halteeinrichtungen (12) gegenseitig auf Abstand haltenden Distanzelement (34, 34', 34'', 34''', 34'''') gebildet ist, wobei das Verbindungselement (20) an einem Ende (36) mit einem Gewinde (38) und an dem anderen Ende (42) mit ebenfalls einem Gewinde (44) oder einer flanschartigen Verdickung (50) zur Festlegung bzw. Verspannung von Halteeinrichtungen (12) und Distanzelement (34, 34', 34'', 34''', 34'''') versehen ist.

2. Vorrichtung nach Anspruch 1,
**dadurch gekennzeichnet**,
daß die Stirnflächen (52) der beiden Enden (54, 56) des wenigstens einen Distanzelements (34) mit der zu der Längsachse des Distanzelements (34) senkrechten Ebene zusammenfallen.

3. Vorrichtung nach Anspruch 1 und/oder 2,
**dadurch gekennzeichnet**,
daß eine der Stirnflächen (52') der beiden Enden (54', 56') des wenigstens einen Distanzelements (34') mit der zu der Längsachse des Distanzelements (34') senkrechten Ebene und eine der Stirnflächen (52') der beiden Enden (54', 56') des wenigstens einen Distanzelements (34') mit einer von der zu der Längsachse des Distanzelements (34') senkrechten Ebene verschiedenen Ebene zusammenfallen.

4. Vorrichtung nach wenigstens einem der Ansprüche 1 bis 3,
**dadurch gekennzeichnet**,
daß die Stirnflächen (52'') der beiden Enden (54'', 56'') des wenigstens einen Distanzelements (34'') mit einer von der zu der Längsachse des Distanzelements (34'') senkrechten Ebene verschiedenen Ebene zusammenfallen.

5. Vorrichtung nach wenigstens einem der Ansprüche 1 bis 4,
**dadurch gekennzeichnet**,
daß das Distanzelement (34, 34', 34'', 34''', 34'''') unterschiedlich lang ausgebildet ist.

6. Vorrichtung nach wenigstens einem der Ansprüche 2 bis 5,
**dadurch gekennzeichnet**,
daß die Stirnflächen (52, 52', 52'', 52''', 52'''') jedes Distanzelelments (34, 34', 34'', 34''', 34'''') zur rotationsstabilen Anlage mit der/den Halteeinrichtung/en (12) und/oder dem/den benachbarten Distanzelementen (34, 34', 34'', 34''', 34'''') aufgerauht sind.

7. Vorrichtung nach wenigstens einem der Ansprüche 1 bis 6,
**dadurch gekennzeichnet**,
daß die Stirnflächen (52, 52', 52'', 52''', 52'''') jedes Distanzelements (34, 34', 34'', 34''', 34'''') zur roationsstabilen Anlage mit der/den Halteeinrichtung/en (12) und/oder dem/den benachbarten Distanzelement/en (34, 34', 34'', 34''', 34'''') mit etwa radial verlaufenden Nuten, Kerben od. dgl. und Vorsprüngen od. dgl. versehen sind.

8. Vorrichtung nach wenigstens einem der Ansprüche 1 bis 7,
**dadurch gekennzeichnet**,
daß das wenigstens eine Distanzelement (34''') einen Außenumfang mit einer Schlüsselfläche zum Verdrehen des Distanzelements (34''') mittels eines Werkzeugs, insbesondere eines Schraubenschlüssels, aufweist.

9. Vorrichtung nach wenigstens einem der Ansprüche 1 bis 8,
**dadurch gekennzeichnet**,
daß ein weiteres stabförmiges, von Halteeinrichtungen (12) aufnehmbares Verbindungselement (60, 68) wenigstens teilweise und im wesentlichen parallel zu dem einen stabförmigen, von den Halteeinrichtungen (12) aufnehmbaren Verbindungselement (20) verlaufend anordnenbar und zu letzterem relativ festlegbar ist.

10. Vorrichtung nach Anspruch 9,
**dadurch gekennzeichnet**,
daß an dem wenigstens einen Distanzelement (34'''') für das Verbindungselement (20) ein weiteres, im wesentlichen parallel verlaufendes Distanzelement (34) für das weitere Verbindungselement (60) unlösbar angebracht, insbesondere angeschweißt, ist.

11. Vorrichtung nach wenigstens einem der Ansprüche 9 und 10,
**dadurch gekennzeichnet**,
daß die beiden stabförmigen, von den Halteeinrichtungen (12) aufnehmbaren Verbindungselemente (20, 68) über Halteelemente (64) miteinander verbunden sind.

12. Vorrichtung nach Anspruch 11,
**dadurch gekennzeichnet**,
daß die Halteelemente (64) mit zwei oder mehreren Ausnehmungen (66) od. dgl. zur etwa spielfreien Aufnahme der beiden stabförmigen Verbindungselemente (20, 68) versehen sind.

13. Vorrichtung nach wenigstens einem der Ansprüche 1 bis 12,
**dadurch gekennzeichnet**,
daß die schraubenförmigen Halteeinrichtungen (12) jeweils einen Schraubenschaft (14) mit einem Gewinde (16) zum Einschrauben in den Wirbel und einen Schraubenkopf (18) zur Aufnahme und Festlegung des stabförmigen Verbindungselements (20) umfassen.

14. Vorrichtung nach Anspruch 13,
**dadurch gekennzeichnet**,
daß der Schraubenkopf (18) der schraubenförmigen Halteeinrichtung (12) eine Bohrung, vorzugsweise eine Mittelbohrung (22), zur etwa spielfreien Aufnahme des stabförmigen Verbindungselements (20) aufweist.

15. Vorrichtung nach Anspruch 13,
**dadurch gekennzeichnet**,
daß der Schraubenkopf (18) der schraubenförmigen Halteeinrichtung (12) mit einem Längsschlitz, vorzugsweise einem Mittellängsschlitz (28), od. dgl. schlitzförmiger Ausnehmung zur etwa spielfreien Aufnahme des stabförmigen Verbindungselements (20) versehen ist.

16. Vorrichtung nach wenigstens einem der Ansprüche 13 bis 15,
**dadurch gekennzeichnet**,
daß der Schraubenkopf (18) und der Schraubenschaft (14) der schraubenförmigen Halteeinrichtung (12) über einen Quersteg (24) und/oder einen Längssteg (26) miteinander verbunden sind.

17. Vorrichtung nach wenigstens einem der Ansprüche 1 bis 16,
**dadurch gekennzeichnet**,
daß die Halteeinrichtungen (12) und/oder das stabförmige Verbindungselement (20, 60, 68) und/oder die mit dem Verbindungselement (20, 60, 68) zusammenwirkenden Muttern (40, 46) aus Metall, insbesondere aus einer Metallegierung bestehen.

18. Vorrichtung nach wenigstens einem der Ansprüche 1 bis 17,
**dadurch gekennzeichnet**,
daß das/die Distanzelement/e (34, 34', 34'', 34''', 34'''') aus Metall, insbesondere aus Titan oder einer Legierung hieraus, besteht/bestehen.

19. Vorrichtung nach wenigstens einem der Ansprüche 1 bis 17,
**dadurch gekennzeichnet**,
daß das/die Distanzelement/e (34, 34', 34'', 34''', 34'''') aus einem bedingt elastischen/flexiblen Material, insbesondere Polyethylen, besteht/bestehen.

## Claims

1. Device for stiffening and/or correcting a section of the spine which consists of at least two vertebrae, the device comprising at least two screw-shaped holding arrangements (12) which can in each case be fastened to one of the vertebrae of the section of the spine for the purpose of receiving and securing a compression or distraction rod (32) which connects the holding arrangements (12) to one another, characterized in that the compression or distraction rod (32) is made up of a rod-shaped connection element (20) which can be received by the holding arrangements (12), and of at least one spacer element (34, 34' 34'', 34''', 34'''') which can be received in a displaceable manner by the rod-shaped connection element (20), is arranged between two adjacent holding arrangements (12) and keeps these holding arrangements (12) at a distance from one another, the connection element (20) being provided at one end (36) with a thread (38) and being provided at the other end (42) likewise with a thread (44) or a flange-like thickening (50) for securing or clamping together the holding arrangements (12) and spacer element (34, 34', 34'', 34''', 34'''').

2. Device according to Claim 1, characterized in that the end faces (52) of the two ends (54, 56) of the at least one spacer element (34) coincide with the plane perpendicular to the longitudinal axis of the spacer element (34).

3. Device according to Claim 1 and/or 2, characterized in that one of the end faces (52') of the two ends (54', 56') of the at least one spacer element (34') coincides with the plane perpendicular to the longitudinal axis of the spacer element (34'), and one of the end faces (52') of the two ends (54', 56') of the at least one spacer element (34') coincides with a plane other than the plane perpendicular to the longitudinal axis of the spacer element (34').

4. Device according to at least one of Claims 1 to 3, characterized in that the end faces (52'') of the two ends (54'', 56'') of the at least one spacer element (34'') coincide with a plane other than the plane perpendicular to the longitudinal axis of the spacer element (34'').

5. Device according to at least one of Claims 1 to 4, characterized in that the spacer element (34, 34', 34'', 34''', 34'''') is designed having a different length.

6. Device according to at least one of Claims 2 to 5, characterized in that the end faces (52, 52', 52'', 52''', 52'''') of each spacer element (34, 34', 34'', 34''', 34'''') are roughened for bearing in a rotationally stable manner on the holding arrangement(s) (12) and/or the adjacent spacer element(s) (34, 34', 34'', 34''', 34'''').

7. Device according to at least one of Claims 1 to 6, characterized in that the end faces (52, 52', 52'', 52''', 52'''') of each spacer element (34, 34', 34'', 34''', 34'''') are provided with approximately radially extending grooves, notches or the like and projections or the like for bearing in a rotationally stable manner on the holding arrangement(s) (12) and/or the adjacent spacer element(s) (34, 34', 34'', 34''', 34'''').

8. Device according to at least one of Claims 1 to 7, characterized in that the at least one spacer element (34''') has an outer circumference with a wrench face for turning the spacer element (34''') by means of a tool, in particular a spanner.

9. Device according to at least one of Claims 1 to 8, characterized in that a further rod-shaped connection element (60, 68) which can be received by holding arrangements (12) can be arranged to extend at least partially and essentially parallel to the one rod-shaped connection element (20) which can be received by the holding arrangements (12), and it can be fastened relative to the connection element (20).

10. Device according to Claim 9, characterized in that on the at least one spacer element (34'''') for the connection element (20), a further, essentially parallel spacer element (34) for the further connection element (60) is arranged unreleasably thereon, in particular welded thereon.

11. Device according to at least one of Claims 9 and 10, characterized in that the two rod-shaped connection elements (20, 68) which can be received by the holding arrangements (12) are connected to one another via holding elements (64).

12. Device according to Claim 11, characterized in that the holding elements (64) are provided with two or more recesses (66) or the like for receiving the two rod-shaped connection elements (20, 68) approximately without play.

13. Device according to at least one of Claims 1 to 12, characterized in that the screw-shaped holding arrangements (12) each comprise a screw shank (14) with a thread (16) for screwing into the vertebra, and a screw head (18) for receiving and securing the rod-shaped connection element (20).

14. Device according to Claim 13, characterized in that the screw head (18) of the screw-shaped holding arrangement (12) has a bore, preferably a central bore (22), for receiving the rod-shaped connection element (20) approximately without play.

15. Device according to Claim 13, characterized in that the screw head (18) of the screw-shaped holding arrangement (12) is provided with a longitudinal slot, preferably a central longitudinal slot (28), or similar slot-shaped recess for receiving the rod-shaped connection element (20) approximately without play.

16. Device according to at least one of Claims 13 to 15, characterized in that the screw head (18) and the screw shank (14) of the screw-shaped holding arrangement (12) are connected to one another via a transverse bridge (24) and/or a longitudinal bridge (26).

17. Device according to at least one of Claims 1 to 16, characterized in that the holding arrangements (12) and/or the rod-shaped connection element (20, 60, 68) and/or the nuts (40, 46) interacting with the connection element (20, 60, 68) are made of metal, in particular of a metal alloy.

18. Device according to at least one of Claims 1 to 17, characterized in that the spacer element(s) (34, 34', 34'', 34''', 34'''') is/are made of metal, in particular of titanium or an alloy thereof.

19. Device according to at least one of Claims 1 to 17, characterized in that the spacer element(s) (34, 34', 34'', 34''', 34'''') is/are made of a limitedly elastic/flexible material, in particular polyethylene.

## Revendications

1. Dispositif permettant de raidir et/ou de corriger une section d'au moins deux vertèbres de la colonne vertébrale, comprenant au moins deux dispositifs de maintien (12) en forme de vis, chacun de ces dispositifs pouvant être fixé à une des vertèbres de la section de la colonne vertébrale, et étant destiné à recevoir et à fixer une tige de compression ou de traction (32) reliant entre eux les dispositifs de maintien (12), caractérisé en ce que la tige de compression ou de traction (32) est constituée d'un élément de raccordement (20) en forme de tige susceptible d'être logé dans les dispositifs de maintien (12), et d'au moins un élément d'écartement (34,34',34'',34''',34'''') susceptible de recevoir mobile en coulissement l'élément de raccordement (20) en forme de tige, élément d'écartement placé chacun entre deux dispositifs de maintien (12) adjacents et maintenant ces dispositifs de maintien (12) à distance l'un de l'autre ; lequel élément de raccordement (20) est pourvu à une extrémité (36) d'un filetage (38) et à une autre extrémité (42) également d'un filetage (44) ou d'un renflement (50) de type flasque et servant à fixer ou à serrer les dispositifs de maintien (12) et l'élément d'écartement (34,34',34'',34''',34'''').

2. Dispositif selon la revendication 1, caractérisé en ce que les faces frontales (52) des deux extrémités (54,56) d'au moins l'un des éléments d'écartement (34) coïncident avec le plan perpendiculaire à l'axe longitudinal de l'élément d'écartement (34).

3. Dispositif selon la revendication 1 et/ou 2, caractérisé en ce que l'une des faces frontales (52') des deux extrémités (54',56') d'au moins l'un des éléments d'écartement (34') coïncide avec le plan perpendiculaire à l'axe longitudinal de l'élément d'écartement (34'), et l'une des faces frontales (52') des deux extrémités (54', 56') d'au moins l'un des éléments d'écartement (34') coïncide avec un plan différent du plan perpendiculaire à l'axe longitudinal de l'élément d'écartement (34').

4. Dispositif selon l'une des revendications 1 à 3, caractérisé en ce que les faces frontales (52'') des deux extrémités (54'',56'') d'au moins l'un des éléments d'écartement (34'') coïncident avec un plan différent du plan perpendiculaire à l'axe longitudinal de l'élément d'écartement (34'').

5. Dispositif selon l'une des revendications 1 à 4, caractérisé en ce que l'élément d'écartement (34,34',34'',34''',34'''') présente des longueurs différentes.

6. Dispositif selon l'une des revendications 2 à 5, caractérisé en ce que les faces frontales (52,52',52'',52''',52'''') de chaque élément d'écartement (34,34',34'',34''',34'''') sont poncées afin que leur installation avec le/les dispositif(s) de maintien (12) et/ou le/les éléments d'écartement (34,34',34'',34''',34'''') voisin(s) soit stable en rotation.

7. Dispositif selon l'une des revendications 1 à 6, caractérisé en ce que les faces frontales (52,52',52'',52''',52'''') de chaque élément d'écartement (34, 34',34'',34''',34'''') sont pourvues d'encoches, d'entailles etc... et de parties en saillies etc... sensiblement radiales, ceci afin que soit assurée la stabilité en rotation de leur installation avec le/les dispositif(s) de maintien (12) et/ou le/les élément(s) d'écartement voisin(s) (34,34',34'',34''',34'''').

8. Dispositif selon l'une des revendications 1 à 7, caractérisé en ce qu'au moins l'un des éléments d'écartement (34''') présente un périmètre extérieur avec un emplacement pour une clé afin de pouvoir tourner l'élément d'écartement (34''') au moyen d'un outil, tout particulièrement d'une clé de serrage.

9. Dispositif selon l'une des revendications 1 à 8, caractérisé en ce qu'un élément supplémentaire de raccordement (60,68) en forme de tige, pouvant être logé dans des dispositifs de maintien (12), peut être agencé au moins partiellement et sensiblement parallèlement à l'un des éléments de raccordement (20) en forme de tige destiné à être logé dans les dispositifs de maintien (12) et peut être fixé par rapport à cet élément de raccordement (20).

10. Dispositif selon la revendication 9, caractérisé en ce qu'un élément d'écartement (34) supplémentaire pour l'élément de raccordement supplémentaire (60), sensiblement parallèle, est rapporté de manière non démontable à au moins l'un des éléments d'écartement (34'''') de l'élément de raccordement (20), en particulier est soudé.

11. Dispositif selon l'une des revendications 9 et 10, caractérisé en ce que les deux éléments de raccordement (20,68) en forme de tige susceptibles d'être logés dans les dispositifs de maintien (12) sont reliés l'un à l'autre par des éléments de maintien (64).

12. Dispositif selon la revendication 11, caractérisé en ce que les éléments de maintien (64) sont pourvus de deux ou de plusieurs échancrures (66), ou analogues, pour recevoir pratiquement sans jeu les deux éléments de raccordement en forme de tige (20,68).

13. Dispositif selon l'une des revendications de 1 à 12, caractérisé en ce que les dispositifs de maintien (12) en forme de vis comportent chacun une tige filetée (14) avec un filetage (16) destiné à être vissé dans la vertèbre, et une tête de vis (18) destinée à loger et fixer l'élément de raccordement (20) en forme de tige.

14. Dispositif selon la revendication 13, caractérisé en ce que la tête de vis (18) du dispositif de maintien (12) en forme de vis comporte un perçage, de préférence un perçage central (22), pour recevoir pratiquement sans jeu l'élément de raccordement (20) en forme de tige.

15. Dispositif selon la revendication 13, caractérisé en ce que la tête de vis (18) du dispositif de maintien (12) en forme de vis est pourvue d'une fente oblongue, qui est de préférence une fente oblongue centrale (28), ou d'une cavité en forme de fente analogue, pour recevoir pratiquement sans jeu l'élément de raccordement (20) en forme de tige.

16. Dispositif selon l'une des revendications 13 à 15, caractérisé en ce que la tête de vis (18) et la tige filetée (14) du dispositif de maintien (12) en forme de vis sont reliées au moyen d'une entretoise transversale (24) et/ou d'une entretoise longitudinale (26).

17. Dispositif selon l'une des revendications 1 à 16, caractérisé en ce que les dispositifs de maintien (12) et/ou l'élément de raccordement (20,60,68) en forme de tige et/ou les écrous (40,46) concourant avec l'élément de raccordement (20,60,68) sont en métal, et tout particulièrement en alliage de métaux.

18. Dispositif selon l'une des revendications 1 à 17, caractérisé en ce que le(s) élément(s) d'écartement (34,34',34'',34''',34'''') est/sont en métal, et tout particulièrement en titane ou dans un alliage de titane.

19. Dispositif selon l'une des revendications 1 à 17, caractérisé en ce que le(s) élément(s) d'écartement (34,34';34'',34''',34'''') est/sont en matériau élastique et flexible consécutif, et tout particulièrement en polyéthylène.
